# EUROPEAN PATENT APPLICATION

(11) **EP 4 748 355 A1**
(43) Date of publication of application: **27.05.2026**
(21) Application number: 24843203.1
(22) Date of filing: 19.07.2024
(51) Int. Cl.: A61F 9/007, A61M 5/303

(54) **INTRAVITREAL ADMINISTRATION TOOL AND NEEDLELESS SYRINGE**

(30) Priority: 20.07.2023 JP 2023118160
(71) Applicant: Daicel Corporation, Osaka-shi, Osaka 530-0011 (JP)
(72) Inventor: YAMAMOTO, Yuzo, Tokyo 108-8230 (JP)
(74) Representative: Grünecker Patent- und Rechtsanwälte PartG mbB
(86) International application number: PCT/JP2024/025975
(87) International publication number: WO 2025/018415

(57) **Abstract**

An intravitreal administration tool (50) is provided which positions a needleless injector that injects a substance to be injected into a vitreous body of an eyeball without using an injection needle. The intravitreal administration tool includes a guide (54), at least a part of which is formed in a shape corresponding to the outer edge of a cornea of the eyeball, and a positioning portion (51) that is connected to the guide (54) and the needleless injector and that positions an ejection port (77), of the needleless injector, from which the substance to be injected is ejected, at a position away from the outer edge of the cornea by a predetermined distance rearward of the eyeball when the guide (54) is disposed in alignment with the outer edge of the cornea.

## Description

### Technical Field

The present invention relates to an intravitreal administration tool and a needleless injector.

### Background Art

An intravitreal injection is used for treatment of age-related macular degeneration, retinal vein occlusion, diabetic macular edema, and the like. In the intravitreal injection, since the cornea or crystalline lens might be damaged if the injection position is too close to the cornea, or the retina might be damaged if the injection position is far from the cornea and too close to the back side of the eyeball, there is a guideline that "the injection needle is to be inserted at a position 3.5 mm to 4.0 mm rearward from the corneal limbus".

In Patent Document 1, a fixing tool for an intravitreal injection is disclosed that is formed of an eyeball fixing tool that includes an injection needle fixing plate having an injection needle insertion hole and a ring having a non-smooth eyeball contact surface and that fixes the position of the injection needle insertion hole on the eyeball.

### Citation List

### Patent Document

Patent Document 1: WO 2007/052730

### Summary of Invention

### Technical Problem

Since the fixing tool for the intravitreal injection disclosed in Patent Document 1 is configured to determine the injection position by inserting the injection needle into the injection needle insertion hole, the fixing tool for the intravitreal injection cannot be applied to a needleless injector. Further, in the case of the fixing tool for the intravitreal injection disclosed in Patent Document 1, a practitioner performs operations of holding the fixing tool for the intravitreal injection with one hand and aligning the ring with the corneal limbus, and holding an injector with the other hand, inserting the injection needle into the eyeball through the injection needle insertion hole, and pushing a plunger to inject a drug solution. Therefore, the practitioner has to perform separate operations with both hands and is required to pay attention to the operation of each hand. Thus, there is a problem that it is difficult to perform the intravitreal injection precisely even when the fixing tool for the intravitreal injection is used.

In view of the above circumstances, an object of the present disclosure is to provide a technique for facilitating an intravitreal injection.

### Solution to Problem

In order to solve the problem described above, an intravitreal administration tool according to the present disclosure is configured to position a needleless injector configured to inject a substance to be injected into a vitreous body of an eyeball without using an injection needle. The intravitreal administration tool includes a guide, at least a part of the guide being formed in a shape corresponding to an outer edge of a cornea of the eyeball, and a positioning portion connected to the guide and the needleless injector and configured to position an ejection port, of the needleless injector, from which the substance to be injected is ejected, at a position away from the outer edge of the cornea by a predetermined distance rearward of the eyeball when the guide is disposed in alignment with the outer edge of the cornea.

In the intravitreal administration tool, the needleless injector may include a housing portion having a housing space in which the substance to be injected is housed, the housing portion including the ejection port from which the substance to be injected is ejected to the target region, and the positioning portion may be attached to the housing portion of the needleless injector.

In the intravitreal administration tool, the needleless injector may include a housing portion having a housing space in which the substance to be injected is housed, the housing portion including the ejection port from which the substance to be injected is ejected to the target region, and a holding portion configured to hold the housing portion, and the positioning portion may be attached to the holding portion of the needleless injector.

In the intravitreal administration tool, the guide may be formed in a curved shape along the outer edge of the cornea.

Further, the positioning portion may position the ejection port of the needleless injector at a position away from the outer edge of the cornea by 3.5 mm to 4.0 mm when the guide is disposed along the outer edge of the cornea.

In the intravitreal administration tool, the guide may be formed in a linear shape in contact with a part of the outer edge of the cornea.

Further, the positioning portion may position the ejection port of the needleless injector at a position away from the outer edge of the cornea by 3.5 mm to 4.0 mm when a linear portion of the guide is disposed along a tangent of the outer edge of the cornea.

In the intravitreal administration tool, the positioning portion may be provided with an irregular portion on a surface facing the eyeball when the guide is disposed along the outer edge of the cornea, the irregular portion being configured to suppress positional misalignment.

In the intravitreal administration tool, the guide may be provided with an irregular portion on a surface facing the eyeball when the guide is disposed along the outer edge of the cornea, the irregular portion being configured to suppress positional misalignment.

In order to solve the problem described above, a needleless injector according to the present disclosure is configured to inject a substance to be injected into a vitreous body of an eyeball without using an injection needle. The needleless injector includes a housing portion having a housing space in which the substance to be injected is housed and an ejection port from which the substance to be injected is ejected to the eyeball, a drive unit configured to provide ejection energy for ejecting the substance to be injected, and an intravitreal administration tool configured to determine an injection position of the needleless injector with respect to the eyeball. The intravitreal administration tool includes a guide, at least a part of the guide being formed in a shape corresponding to an outer edge of a cornea of the eyeball, and a positioning portion connected to the guide and the needleless injector and configured to position the ejection port at a position away from the outer edge of the cornea by a predetermined distance rearward of the eyeball when the guide is disposed in alignment with the outer edge of the cornea.

In the needleless injector, the positioning portion of the intravitreal administration tool may be attached to the housing portion.

The needleless injector may further include a holding portion configured to hold the housing portion, and the positioning portion of the intravitreal administration tool may be attached to the holding portion.

In the needleless injector, the guide may be formed in a curved shape along the outer edge of the cornea.

Further, the positioning portion may position the ejection port of the needleless injector at a position away from the outer edge of the cornea by 3.5 mm to 4.0 mm when the guide is disposed along the outer edge of the cornea.

In the needleless injector, the guide may be formed in a linear shape in contact with a part of the outer edge of the cornea. Further, the positioning portion may position the ejection port of the needleless injector at a position away from the outer edge of the cornea by 3.5 mm to 4.0 mm when a linear portion of the guide is disposed along a tangent of the outer edge of the cornea.

In the needleless injector, the positioning portion may be provided with an irregular portion on a surface facing the eyeball when the guide is disposed along the outer edge of the cornea, the irregular portion being configured to suppress positional misalignment.

In the needleless injector, the guide may be provided with an irregular portion on a surface facing the eyeball when the guide is disposed along the outer edge of the cornea, the irregular portion being configured to suppress positional misalignment.

### Advantageous Effects of Invention

According to the present disclosure, a technique for facilitating an intravitreal injection can be provided.

### Brief Description of Drawings

FIG. 1 is a diagram schematically illustrating the appearance of an injector.
FIG. 2 is a first cross-sectional view of the injector, which is an AA cross section in FIG. 4 described below.
FIG. 3 is a second cross-sectional view of the injector, which is a BB cross section in FIG. 4 described below. The BB cross section is orthogonal to the AA cross section.
FIG. 4 is a diagram illustrating a configuration of a housing that forms an injector 1.
FIG. 5 is a diagram illustrating a schematic configuration of an injector assembly.
FIG. 6 is a cross-sectional view illustrating a schematic configuration of a container.
FIG. 7 is an external view illustrating the schematic configuration of the container.
FIG. 8 is a diagram illustrating a schematic configuration of an intravitreal administration tool, and is a view (side view) of the intravitreal administration tool as viewed from a direction orthogonal to an ejection direction of the injector.
FIG. 9 is a cross-sectional view of FIG. 8.
FIG. 10 is a view of the intravitreal administration tool as viewed from a distal end side.
FIG. 11 is a view of the intravitreal administration tool as viewed from a base end side.
FIG. 12 is a perspective view illustrating a schematic configuration of the intravitreal administration tool.
FIG. 13A is a diagram illustrating a state in which a guide is disposed in a manner that an interval between a guide portion and the outer edge of the cornea is uniform.
FIG. 13B is a diagram illustrating a state in which the guide is disposed in a manner that a portion of the guide portion closest to a positioning portion coincides with the outer edge of the cornea.
FIG. 14 is a schematic explanatory view illustrating an injection position when a guide is disposed in alignment with the outer edge of the cornea.
FIG. 15 is a diagram illustrating a schematic configuration of an intravitreal administration tool and a container according to a second embodiment.
FIG. 16 is a diagram illustrating a schematic configuration of an intravitreal administration tool and a container according to a third embodiment.
FIG. 17 is a diagram for describing a positional relationship between the container and a container holder according to the third embodiment.
FIG. 18 is a cross-sectional view illustrating a schematic configuration of an intravitreal administration tool and a container according to a fourth embodiment.
FIG. 19 is a diagram illustrating the intravitreal administration tool according to the fourth embodiment as viewed from the distal end side.
FIG. 20 is a diagram illustrating a schematic configuration of an intravitreal administration tool according to a fifth embodiment.
FIG. 21 is a schematic explanatory view illustrating the injection position when the intravitreal administration tool is disposed in alignment with the outer edge of the cornea.
FIG. 22 is a diagram illustrating a modified example in which the guide is formed having a length in the circumferential direction shorter than that in the example of FIG. 20.
FIG. 23 is a diagram illustrating examples of a guide including a linear guide portion.
FIG. 24 is a diagram illustrating examples in which the guide portions illustrated in FIG. 23 are disposed in alignment with the outer edge of the cornea.
FIG. 25 is a diagram illustrating an example in which a circular guide portion is disposed in alignment with the outer edge of the cornea.
FIG. 26 is a diagram illustrating an example of a guide 54F in which a length measurement starting point is set at a point other than a point of contact between the guide portion and the outer edge of the cornea.
FIG. 27 is a view illustrating an example of a guide portion having a curved shape that is protruding toward the cornea side.
FIG. 28 is a diagram illustrating an example in which a circular guide portion is disposed surrounding the injection position.
FIG. 29 is a diagram illustrating an example of a guide portion obtained by combining a straight line and a curved line.

### Description of Embodiments

### First Embodiment

With reference to the drawings, a needleless injector 1 according to an embodiment of the present disclosure (herein, simply referred to as "injector") is described below. The injector 1 is a needleless injector that ejects an ejection solution, which corresponds to a substance to be injected of the present application, to an eyeball (target region), using combustion energy of an explosive. In other words, the injector 1 is a device that performs an injection by ejecting the ejection solution to the target region without using an injection needle.

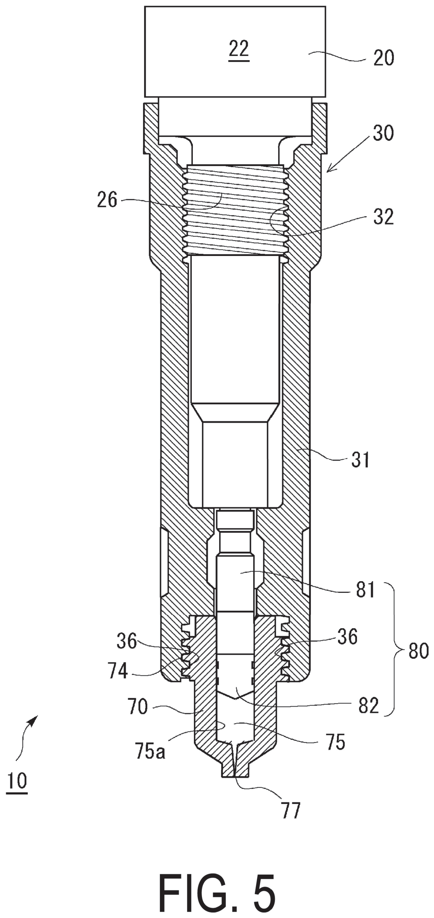

Note that each of the configurations, combinations thereof, and the like in each embodiment are examples, and various additions, omissions, substitutions, and other changes may be made as appropriate without departing from the spirit of the present disclosure. The present disclosure is not limited by the embodiments and is limited only by the claims. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

### Configuration of Injector 1

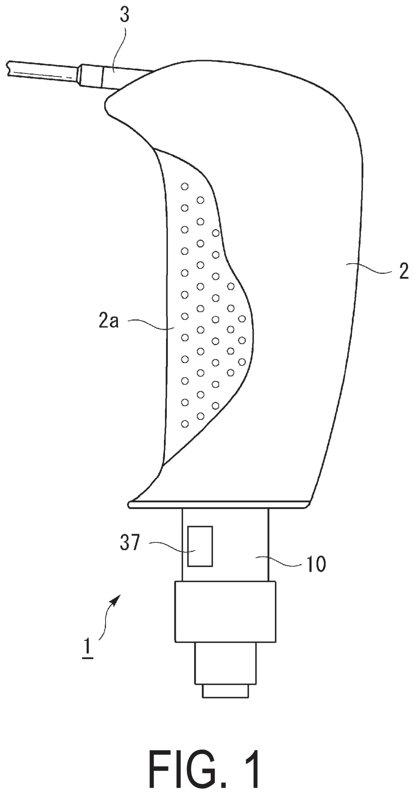

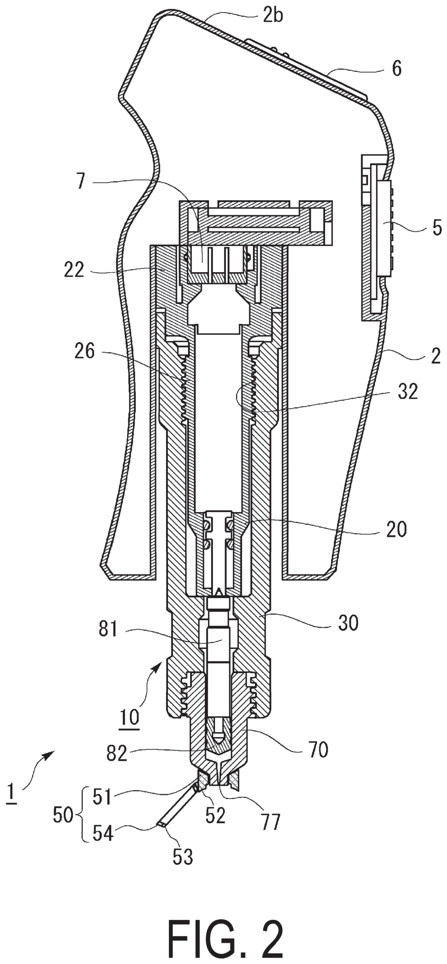

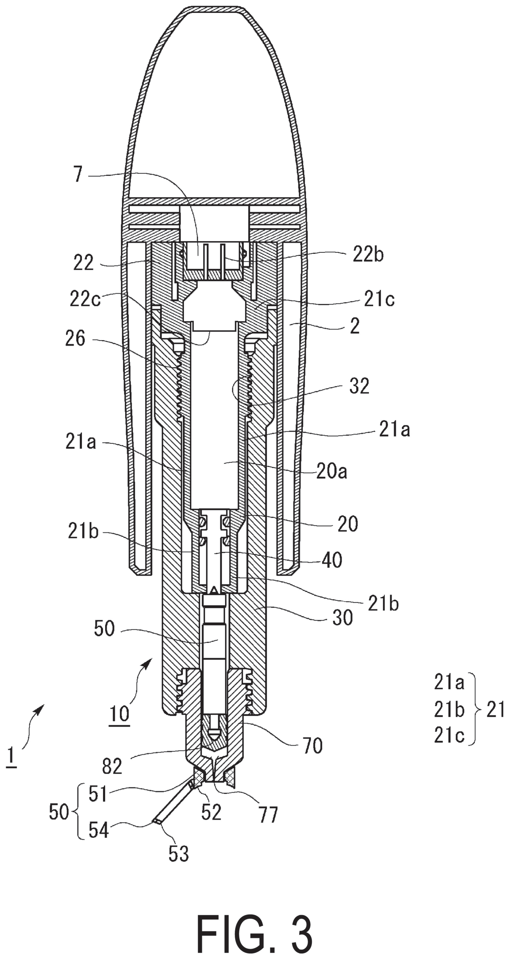

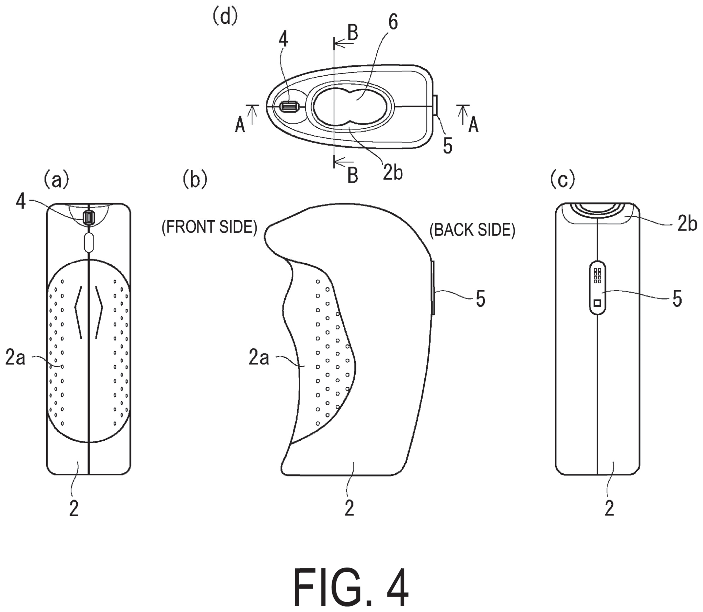

Here, FIG. 1 is a diagram schematically illustrating the appearance of the injector 1. FIG. 2 is a first cross-sectional view of the injector 1, which is an AA cross section in FIG. 4 described later. FIG. 3 is a second cross-sectional view of the injector 1, which is a BB cross section in FIG. 4 described later. The BB cross section is orthogonal to the AA cross section. FIG. 4 is a diagram illustrating a configuration of the housing 2 that is included in the injector 1, and FIG. 5 is a diagram illustrating a schematic configuration of the injector assembly 10. The injector 1 is formed by the injector assembly 10 being attached to the housing 2. A power cable 3 (FIG. 1) for supplying a drive current to the igniter 22 in the injector assembly 10 is connected to the housing 2.

Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed instead of being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid being the medium of the ejection solution is only required to be a substance suitable for administering the predetermined substance exemplified by those substances to the target region, and may be aqueous or oleaginous, which is not limited. Further, viscosity of the liquid being the medium is not particularly limited as long as the predetermined substance can be ejected by the injector 1.

In the injector 1, the injector assembly 10 is configured to be attachable to and detachable from the housing 2 freely. A housing space 75 (see FIG. 5) formed between a container (housing portion) 70 and a plunger 80 in the injector assembly 10 is filled with the ejection solution during a preparation stage, which is a stage before the operation of the injector 1. The injector assembly 10 is a unit that is replaced each time the ejection solution is ejected. The injector assembly 10 will be described in detail below.

On the other hand, the housing 2 has a grip portion 2a formed to be gripped by a user of the injector 1 in use, and is provided with a plurality of switches for operating the injector 1 to eject the ejection solution. Note that the injector 1 can be held and operated by one hand of the user. In this context, the housing 2 will be described with reference to FIG. 4. In FIG. 4, (a) illustrates the appearance of the housing 2 as viewed from the front side, (b) illustrates the appearance of the housing 2 as viewed from one side, (c) illustrates the appearance of the housing 2 as viewed from the back side, and (d) illustrates the appearance of the housing 2 as viewed from the upper side. Here, "front side" indicates a portion positioned on the distal side of the user holding the housing 2, which is the left side in FIG. 4(b), and "back side" indicates a portion positioned on the proximal side of the user holding the housing 2, which is the right side in FIG. 4(b). Thus, when the user holds the housing 2 with one hand, fingertips rest on the front side of the distal housing 2, which is the distal side, and the wrist is in the vicinity of the back side of the housing 2, which is the proximal side. The "upper side" is a portion of the injector 1 on the base end side.

Considering such a way of being held by the user, the grip portion 2a is provided at a front side portion of the housing 2 so that the user can easily rest his or her fingertips thereon. The grip portion 2a is provided with a plurality of dimples making the user's fingertips even easier to rest thereon. Furthermore, the grip portion 2a has gentle recesses and protrusions on the front side of its outer shell (see FIG. 4(b)) so that the user's forefinger and middle finger can be easily rested thereon, for the sake of more stable holding of the housing by the user.

Further, the housing 2 is provided with a first switch 5 and a second switch 6 that are two operating switches for operating the injector 1. The first switch 5 and the second switch 6 are connected to a control unit, such as a microcomputer (not illustrated). The control unit controls the supply of ignition current to the igniter 22 based on a signal from each switch, thereby controlling an operation of the injector 1. The first switch 5 is a sliding switch provided on the back side of the housing 2, the sliding direction of which being an upward and downward direction of the housing 2 (direction between the distal end and the base end). The first switch 5 is constantly biased in the upward direction. The user can achieve a standby state of the injector 1 by continuously sliding the first switch 5 downward (toward the distal end side) for a predetermined period of time against the biasing force. The standby state is a state in which the injector 1 is ready to eject the ejection solution. When a user makes an additional operation in this state, the ejection is implemented.

The second switch 6 is a press type switch provided on an inclined surface 2b on the upper side of the housing 2. The user can press the second switch 6 in a direction toward the inner side of the housing 2. The control unit is configured to supply an ignition current to the igniter 22 in response to the pressing operation on the second switch 6 while the injector 1 is in the standby state as a result of the operation on the first switch 5 described above. A connector 4 to which the power cable 3 is connected is provided on the front side of the inclined surface 2b on the upper side of the housing 2. In the present embodiment, the connector 4 is a USB connector, and the power cable 3 is freely attachable to and detachable from the housing 2.

Note that, as described above, in the present embodiment, the power for actuating the igniter 22 is supplied from the outside through the power cable 3. Alternatively, a battery for supplying such power may be provided inside the housing 2. In this case, the housing 2 can be repeatedly used while replacing the injector assembly 10, until the battery runs out. When the battery runs out, the battery may be replaced.

A schematic configuration of the injector assembly 10 is illustrated in FIG. 5. The injector assembly 10 is attached to the housing 2 to form the injector 1, as illustrated in FIGS. 2 and 3. Specifically, the injector assembly 10 is an assembly including an actuator (drive unit) 20, an attachment 30, an intravitreal administration tool 50, the container 70, and the plunger 80. How the injector assembly 10 is assembled will be described below.

The actuator 20 has a body 21 formed in a cylindrical shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. Here, the igniter 22, which is an electric igniter that generates ejection energy through combustion of an ignition agent, is attached to the base end portion 21c of the body 21. The igniter 22 has an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition agent is an energy for the injector 1 to eject the ejection solution to the target region. Note that, examples of the ignition agent include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), and an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. Note that an explosive other than these may be used as the ignition agent as long as appropriate ejection of the ejection solution can be performed.

The body 21 is a cylindrical member, and the internal space of the center portion 21a is a combustion chamber 20a. Furthermore, a male thread portion 26 is formed in a part of the outer surface of the center portion 21a. The male thread portion 26 is configured to be screwed into a female thread portion 32 of the attachment 30 described below. The effective lengths of the male thread portion 26 and the female thread portion 32 are determined in a manner that sufficient coupling force can be guaranteed therebetween. The internal space of the distal end portion 21b adjacent to the center portion 21a is formed in a cylindrical shape in which a piston 40 is slidably disposed.

Then, when the igniter 22 is actuated, the combustion product is discharged into the combustion chamber 20a, the pressure therein rises, and the piston 40 receives the pressure and results in sliding toward the distal end side. Thus, the actuator 20 has a mechanism with the igniter 22 serving as an actuation source and the piston 40 serving as an output unit.

The attachment 30 is a member for attaching the actuator 20, the plunger 80, and the container 70 as illustrated in FIG. 5. For a body 31 of the attachment 30, a resin such as nylon 6-12, polyarylate, polybutylene terephthalate, polyphenylene sulfide, or a liquid crystal polymer, which are known, may be used, for example. Further, filler such as glass fibers and glass filler may be contained in those resins. 20 to 80 mass% of glass fibers may be contained in polybutylene terephthalate, 20 to 80 mass% of glass fibers may be contained in polyphenylene sulfide, or 20 to 80 mass% of minerals may be contained in a liquid crystal polymer. Note that the material of the attachment 30 is not limited to resin, and may be metal or ceramic.

Of the internal space of the body 31, in a region extending from the base end side to the center, the actuator 20 is disposed as illustrated in FIG. 5. Then, of the internal space where the actuator 20 is disposed, in a region on the base end side, the base end portion 21c of the actuator 20 is generally positioned, and in a region on the distal end side whose diameter is smaller than that of the base end side, the center portion 21a and the distal end portion 21b of the actuator 20 are generally positioned. Further, the female thread portion 32 is disposed on the inner wall surface of the body 31, and the female thread portion 32 is formed in a manner that the male thread portion 26 provided at the center portion 21a of the actuator 20 can be screwed into the female thread portion 32.

Further, of the internal space of the body 31, in a region on the distal end side with respect to the region where the actuator 20 is disposed, the plunger 80 is generally disposed as illustrated in FIG. 5. The diameter of the region where the plunger 80 is disposed is smaller than the diameter of the region where the distal end portion 21b of the actuator 20 is disposed, and is a diameter that allows the plunger 80 to slide.

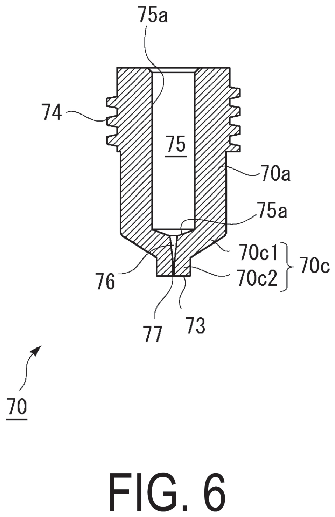

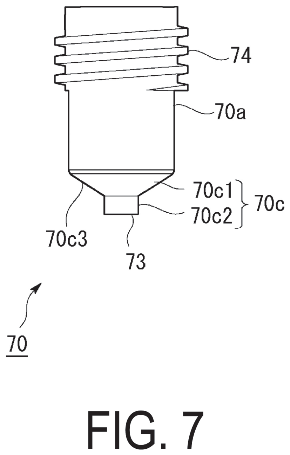

Further, of the internal space of the body 31, in a region on the distal end side, a part of the container 70 is generally disposed. The region where the container 70 is disposed communicates with the region where the plunger 80 is disposed on the base end side of the region, and the distal end side of the region where the container 70 is disposed is open to the distal end surface of the attachment 30. A female thread portion 36 for attaching the container 70 is formed at the inner wall of the region where the container 70 is disposed. A male thread portion 74 of the container 70 illustrated in FIGS. 6 and 7 described below is screwed into the female thread portion 36, whereby the attachment 30 and the container 70 are coupled to each other.

Next, the plunger 80 will be described. The plunger 80 is a member that pressurizes the ejection solution using an energy received from the piston 40, and includes a plunger rod 81 and a stopper portion 82. The plunger rod 81 is formed using, for example, a resin material suitable for pressurizing the plunger rod 81, but is not limited thereto, and may be formed using, for example, the same type of material as that of the attachment 30.

The stopper portion 82 formed using an elastic member such as rubber is attached to the distal end side of the plunger rod 81. Note that specific examples of the material of the stopper portion 82 include butyl rubber and silicon rubber. Further, there may be exemplified a styrene-based elastomer, a hydrogenated styrene-based elastomer, or a substance obtained by mixing a styrene-based elastomer or a hydrogenated styrene-based elastomer with polyolefin such as polyethylene, polypropylene, polybutene, or an α-olefin copolymer, oil such as liquid paraffin or process oil, or a powder inorganic substance such as talc, cast, or mica. Further, as the material of the stopper portion 82, a polyvinyl chloride-based elastomer, an olefin-based elastomer, a polyester-based elastomer, a polyamide-based elastomer, a polyurethane-based elastomer, any of various rubber materials (particularly, a vulcanized material) such as natural rubber, isoprene rubber, chloroprene rubber, nitrile butadiene rubber, and styrene butadiene rubber, or a mixture thereof may be employed. Furthermore, the stopper portion 82 pressurizes the ejection solution by sliding inside the container 70 described below. Thus, a surface of the stopper portion 82 and an inner wall surface 75a of the container 70 may be coated or processed using any of various substances, to secure and adjust slidability between the stopper portion 82 and the inner wall surface 75a of the housing space 75 of the container 70. Examples of the coating agent that can be used include polytetrafluoroethylene (PTFE), silicone oil, diamond-like carbon, nanodiamond, and the like.

FIG. 6 is a cross-sectional view illustrating a schematic configuration of the container 70, and FIG. 7 is an external view illustrating the schematic configuration of the container 70. The container 70 is a member that houses the ejection solution and defines a flow path for ejecting, to the target region, the ejection solution pressurized by the plunger 80. The container 70 is formed using a material selected in consideration of the pressurization by the plunger and the definition of the flow path. The container 70 of the present embodiment is formed using a resin material. Note that the container 70 is not limited to being formed using the resin material, and may be formed using, for example, the same type of material as that of the attachment 30.

The container 70 includes the housing space 75 that can house the ejection solution and in which the stopper portion 82 of the plunger 80 can move, and a flow path 76 that connects the housing space 75 to the ejection port 77 facing the outside of the container 70. Specifically, the container 70 includes a body portion 70a having a cylindrical shape and defining the housing space 75, and a nozzle portion 70c that is connected to the distal end side of the body portion 70a and defines the flow path 76. Further, the nozzle portion 70c includes a distal end portion 70c2 internally including a part of the flow path 76 on the ejection port 77 side, and a tapered portion 70c1 that connects the distal end portion 70c2 and the body portion 70a to each other and includes a distal end side outer surface 70c3 inclined with respect to the center axis in the longitudinal direction of the attachment 30. Then, the intravitreal administration tool 50 described below is externally fitted to the outer periphery of the distal end portion 70c2.

In the injector assembly 10, as illustrated in FIG. 5, the distal end side of the plunger 80 is fitted into the housing space 75 of the container 70 in a manner that the stopper portion 82 of the plunger 80 can slide inside the housing space 75 in a direction toward the nozzle portion 70c (direction toward the distal end side). In a state where the plunger 80 is fitted into the container 70, a space formed between the stopper portion 82 of the plunger 80 and the container 70 becomes a storage space of the ejection solution, that is, a space in which the ejection solution is encapsulated. In other words, when the plunger 80 is fitted into the initial position of the housing space 75 in the container 70, the distal end surface of the stopper portion 82 and the inner wall surface, of the container 70, located closer to the distal end side than the distal end surface of the stopper portion 82 define the storage space. The flow path of the container 70 opens in a distal end surface 73 of the nozzle portion 70c to form the ejection port 77. Therefore, when the plunger 80 slides inside the housing space 75, the ejection solution housed in the housing space 75 is pressurized and the ejection solution is ejected from the ejection port 77 through the flow path 76.

The flow path 76 provided in the container 70 has an inner diameter smaller than that of the housing space 75. With this configuration, the ejection solution that has been applied with a high pressure is ejected to the outside through the ejection port 77. Further, the male thread portion 74 for attaching the container 70 to the attachment 30 is formed on the outer periphery on the base end side of the container 70. The male thread portion 74 can be screwed into the female thread portion 36 of the attachment 30.

The distal end shape of the stopper portion 82 of the plunger 80 is formed generally matching the distal end shape of the housing space 75, which is defined by the inner wall surface 75a near a portion at which the housing space 75 and the flow path 76 are connected to each other (the deepest part of the housing space 75). In the present embodiment, both the distal end shape of the stopper portion 82 and the distal end shape of the housing space 75 are tapered shapes that decrease in diameter toward the distal end side. With this configuration, a smallest possible gap can be formed between the stopper portion 82 and the inner wall surface 75a of the container 70 when the plunger 80 slides for ejecting the ejection solution and reaches the deepest part of the housing space 75, whereby the ejection solution can be prevented from wastefully remaining in the housing space 75. However, the shape of the stopper portion 82 is not limited to a particular shape as long as desired effects can be obtained with the injector 1 of the present embodiment. Further, the stopper portion 82 is formed having an outer diameter slightly larger than that of the housing space 75 of the container 70, and thus, when the stopper portion 82 is fitted into the housing space 75 in a state of being compressed in the radial direction, the stopper portion 82 suitably comes into contact with the inner wall surface 75a in a manner that the airtightness is maintained between the stopper portion 82 and the container 70. Note that the stopper portion 82 need not necessarily be formed having a larger outer diameter than the housing space 75 and, for example, may have substantially the same diameter as the housing space 75, as long as the ejection solution can be appropriately ejected.

### Assembly of Injector 1

With regard to the assembly of the injector 1, first, how the injector assembly 10 is assembled will be described. In a state where the stopper portion 82 of the plunger 80 is inserted to the deepest part of the housing space 75 of the container 70, the plunger 80 is retracted with the ejection port 77 of the container 70 in communication with the ejection solution. Since the stopper portion 82 and the inner wall surface 75a of the housing space 75 are in close contact with each other, the retraction action produces a negative pressure in the housing space. Thus, the housing space 75 can be filled with the ejection solution through the ejection port 77. At this time, the plunger 80 is retracted to an extent enough for making the part of the plunger 80 protruding from the container 70 (plunger rod 81) reach the region where the piston 40 is disposed in the internal space of the attachment 30, when the container 70 is attached to the attachment 30 in this state.

When the container 70 with the housing space 75 filled with the ejection solution is attached to the attachment 30, the actuator 20 is further inserted into the attachment 30 from the base end side. The actuator 20 is inserted until the distal end surface of the piston 40 disposed at the distal end portion 21b of the actuator 20 reaches the base end surface of the plunger 80 inside the attachment 30. At this time, the male thread portion 26 provided at the center portion 21a of the actuator 20 is screwed into the female thread portion 32 of the attachment 30, whereby the actuator 20 and the attachment 30 are suitably coupled to each other. At this time, the piston 40 incorporated in the actuator 20 is connected to the plunger 80. Note that the connection between the piston 40 and the plunger 80 is not limited to simple abutment, and fitting portions may be provided at the distal end of the piston 40 and the base end of the plunger 80 and fitted to each other.

When the actuator 20 is attached to the attachment 30 to which the container 70 and the plunger 80 are attached as described above, the plunger 80 is pushed in a manner that it moves from the piston 40 toward the distal end side, whereby the plunger 80 is positioned at a predetermined position (pre-operation position) inside the container 70. At this time, in accordance with the pushing of the plunger 80, the excess ejection solution in the container 70 is discharged from the ejection port 77, and a predetermined amount of the ejection solution remains in the container 70. Note that the volume of the space for housing the ejection solution that is formed between the plunger 80 positioned at the pre-operation position and the container 70 is determined and set to a volume that secures an appropriate ejection amount of the ejection solution when the injector 1 is operated. Therefore, when the injector assembly 10 is assembled as described above, an amount of the ejection solution housed in the housing space 75 of the container 70, that is, an amount of the ejection solution to be ejected is set to the predetermined amount determined in advance.

The injector assembly 10 having the above-described configuration can be loaded into the housing 2 with the ignition pin 22b of the igniter 22 fitted into the socket 7 on the housing 2 side. As a result, the usable injector 1 is prepared (see FIGS. 1 to 3). The user holds the housing 2 of the injector 1 with one hand and slides the first switch 5 located on the back side of the housing 2 for a predetermined period of time, putting the injector 1 in the standby state. Then, the user positions the injector 1 with respect to the eyeball using the intravitreal administration tool 50 as described later, and brings the ejection port 77 into contact with the target region. In this state, when the user presses the second switch 6, the igniter 22 is actuated, and the ejection solution is pressurized via the piston 40 and the plunger 80. Then, the ejection is implemented, and the ejection solution is injected into the target region.

### Configuration of Intravitreal Administration Tool

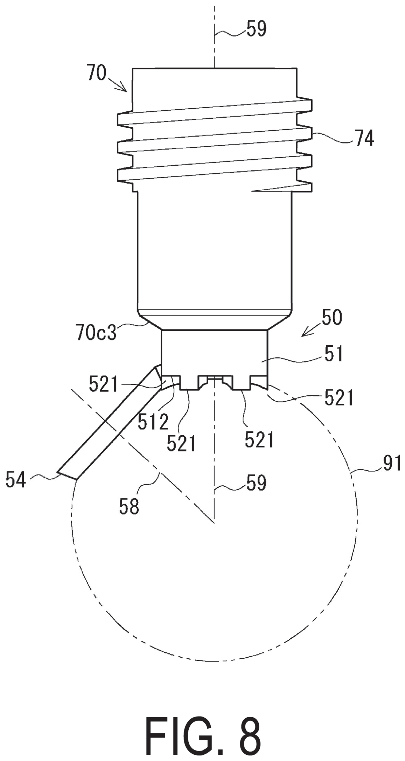

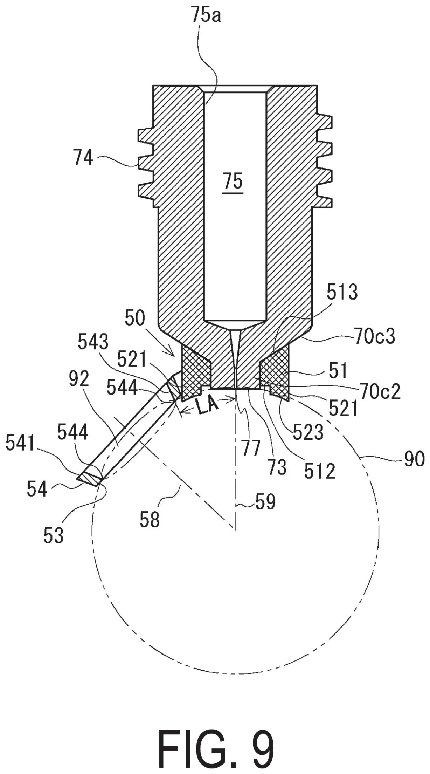

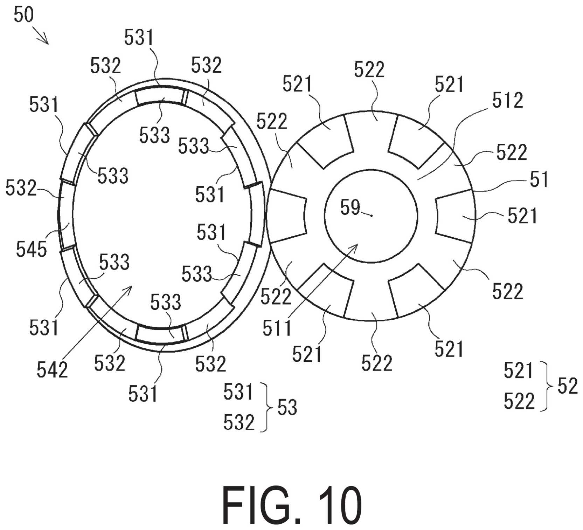

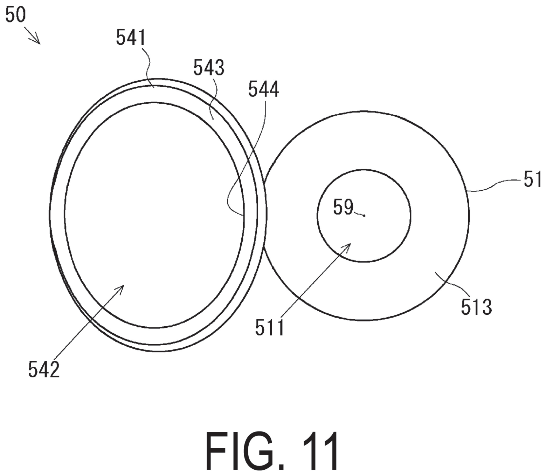

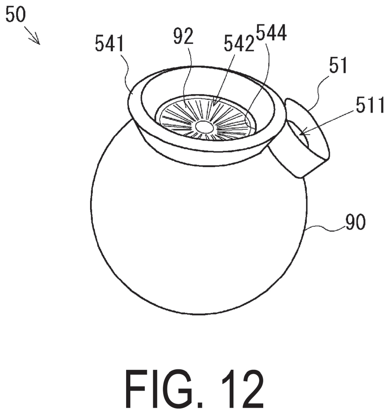

Next, the intravitreal administration tool 50 provided at the distal end of the injector 1 will be described. FIG. 8 is a diagram illustrating a schematic configuration of the intravitreal administration tool 50, and is a view (side view) of the intravitreal administration tool 50 as viewed from a direction orthogonal to an ejection direction of the injector 1. FIG. 9 is a cross-sectional view of FIG. 8, FIG. 10 is a view of the intravitreal administration tool 50 as viewed from the distal end side, FIG. 11 is a view of the intravitreal administration tool 50 as viewed from the base end side, and FIG. 12 is a perspective view illustrating the schematic configuration of the intravitreal administration tool 50.

The intravitreal administration tool 50 includes a guide 54 at least a part of which is formed in a curved shape along the outer edge of the cornea of the eyeball, a positioning portion 51 connected to the guide 54 and the injector 1, and an irregular portion 52 provided on the eyeball side of the positioning portion 51. Note that, in the present embodiment, the guide 54 is formed along the outer edge of the cornea, but since the outer edge of the cornea substantially coincides with the outer edge of the iris when the eyeball is viewed from the front, the outer edge of the cornea may be interpreted as the outer edge of the iris (black eye) in the following description. That is, the guide 54 may be formed with at least a part thereof being along the outer edge of the iris.

The positioning portion 51 is an annular member and is externally fitted to the distal end portion 70c2 of the nozzle portion 70c by the distal end portion 70c2 passing through an inner space 511 of the positioning portion 51. That is, the intravitreal administration tool 50 of the present embodiment is attached to the container 70 of the injector 1. Since the inner diameter of the positioning portion 51, that is, the outer diameter of the inner space 511 is set to be substantially the same as or slightly smaller than the outer diameter of the distal end portion 70c2, when the positioning portion 51 is externally fitted to the distal end portion 70c2, the positioning portion 51 is tightly fitted to the distal end portion 70c2 without having any allowance therebetween. The positioning portion 51 may be detachably attached to the distal end portion 70c2, or may be fixedly attached thereto by adhesion, welding, or the like. The positioning portion 51 of the present embodiment has a circular shape surrounding the distal end portion 70c2. The positioning portion 51 is formed with the position of the center axis of the circle, that is, a center axis 59 of the positioning portion 51 extending along the ejection direction, coinciding with the position of the ejection port 77. Further, in the present embodiment, the ejection port 77 is provided on the center axis of the distal end portion 70c2, and the positioning portion 51 is coaxially attached to the distal end portion 70c2. Note that the positioning portion 51 is not limited to having the circular shape, and may have a shape such as a C shape in which a part of a circle is missing. Note that the intravitreal administration tool 50 of the present embodiment is formed separately from the container 70, but may be formed integrally with the container 70. In this case, the positioning portion 51 is not limited to having the annular shape, and may have any shape as long as the shape allows the container 70 and the guide 54 to be connected to each other.

A surface (base end surface) 513 on the base end side of the positioning portion 51 is formed in a tapered shape along the distal end side outer surface (tapered surface) 70c3 of the tapered portion 70c1 of the nozzle portion 70c. Accordingly, when the positioning portion 51 is pressed against the target region during use of the injector 1, the base end surface 513 comes into contact with and is received by the distal end side outer surface 70c3, and the positional relationship between the intravitreal administration tool 50 and the injector 1 in the ejection direction is maintained with high accuracy. Further, a surface on the distal end side of the positioning portion 51, that is, a surface (base surface) 512 facing the eyeball during use is provided with the irregular portion (anti-slip portion) 52 that suppresses positional misalignment with respect to the eyeball.

The irregular portion 52 includes a plurality of protruding portions 521, and each of the protruding portions 521 is erected on the base surface 512 of the positioning portion 51. The plurality of protruding portions 521 are provided at intervals in the circumferential direction of the base surface 512. Accordingly, a recessed portion 522 is formed between the protruding portions 521. Note that, in the present embodiment, the intervals between the corresponding protruding portions 521 are equal, and the protruding portions 521 are arranged in rotational symmetry about the axis 59.

The guide 54 is disposed in contact with or in proximity to the eyeball during use of the injector 1 and determines the position of the injector 1 with reference to the cornea of the eyeball, which is the target region. For example, a side of the guide 54 that comes into contact with or approaches the eyeball during use is referred to as a distal end side, and a side of the guide 54 that is far from the eyeball in the normal direction of the eyeball is referred to as a base end side. The guide 54 includes an annular peripheral wall 541, and an internal space 542 penetrating the guide 54 from the distal end to the base end thereof is provided inside the peripheral wall 541. The distal end of an inner peripheral surface 543 of the peripheral wall 541 defining the internal space 542 is formed as a guide portion 544, which is formed along the outer edge of the cornea of the eyeball. The guide portion 544 of the present embodiment has a circular shape. That is, the guide 54 of the present embodiment includes the peripheral wall 541 formed in an annular shape, and thus the guide portion 544 has the circular shape. Reference sign 58 indicates the center axis of the circle formed by the peripheral wall 541. In the present embodiment, the distal end side inner edge of the peripheral wall 541 is the guide portion 544, but the distal end side outer edge of the peripheral wall 541 may be formed as the guide portion 544. That is, the guide 54 may be formed with the distal end side outer edge (guide portion 544) of the peripheral wall 541 being along the outer edge of the cornea during use of the injector 1. In this case, the guide 54 is not limited to having the annular shape, and may have a disk shape or a dome shape that covers the cornea.

The material of the guide 54 is not particularly limited. The guide 54 may be formed of, for example, resin, metal, ceramic, or the like. Further, the guide 54 may be formed of a transparent material. In this case, the guide 54 may be formed of a translucent or colored transparent material, and thus the guide portion 544 becomes noticeable. Further, when at least a part of the guide 54 is a transparent member, the guide portion 544 need not necessarily be the edge of the guide 54, and may be a line marked on the transparent member, for example, and may be formed with the line being aligned with the outer edge of the cornea.

In FIGS. 8 and 9, Reference sign 90 indicates a region where the eyeball, which is an injection target, is located during use of the injector 1. Reference sign 92 indicates the position of the cornea of the eyeball. The guide 54 and the positioning portion 51 of the intravitreal administration tool 50 are connected to each other in a manner that they have a predetermined positional relationship. The axis 59 of the positioning portion 51 is provided at a position away from the guide portion 544 of the guide 54 by a predetermined distance (interval) LA. Here, the predetermined distance LA is, for example, 3.5 mm to 4.0 mm. Note that the predetermined distance LA is, for example, a distance (shortest distance) of a straight line connecting the axis 59 and a portion of the guide portion 544 closest to the axis 59. Further, the predetermined distance LA may be the shortest distance between the guide portion 544 and the axis 59 when the distance is measured along a spherical surface corresponding to the eyeball.

Further, the intravitreal administration tool 50 is formed with the center axis 59 of the positioning portion 51 being at a predetermined angle with respect to the guide 54. For example, when the guide 54 is disposed along the outer edge of the cornea, the positioning portion 51 is formed with the center axis 59 facing the ejection direction of the ejection solution. Specifically, in this case, the positioning portion 51 is formed with the center axis 59 passing through the center of the eyeball or the back side (retina side) relative to the center of the eyeball.

A surface of the guide 54 on the distal end side, that is, a surface (base surface) 545 facing the eyeball when the guide 54 is disposed along the outer edge of the cornea is provided with an irregular portion (anti-slip portion) 53 that suppresses the positional misalignment with respect to the eyeball. The irregular portion 53 of the guide 54 includes a plurality of protruding portions 531, and each of the protruding portions 531 is erected on the base surface 545 of the guide 54, as with the irregular portion of the positioning portion 51 described above. The plurality of protruding portions 531 are provided at intervals in the circumferential direction of the base surface 545. Accordingly, a recessed portion 532 is formed between the protruding portions 531. Note that, in the present embodiment, the intervals between the corresponding protruding portions 521 are equal, and the protruding portions 531 are arranged in rotational symmetry about an axis 58. Note that the irregular portions 52, 53 are not essential components and may be omitted as appropriate. For example, only the positioning portion 51 may be provided with the irregular portion 52 and the irregular portion 53 of the guide 54 may be omitted, or only the guide 54 may be provided with the irregular portion 53 and the irregular portion 52 of the positioning portion 51 may be omitted.

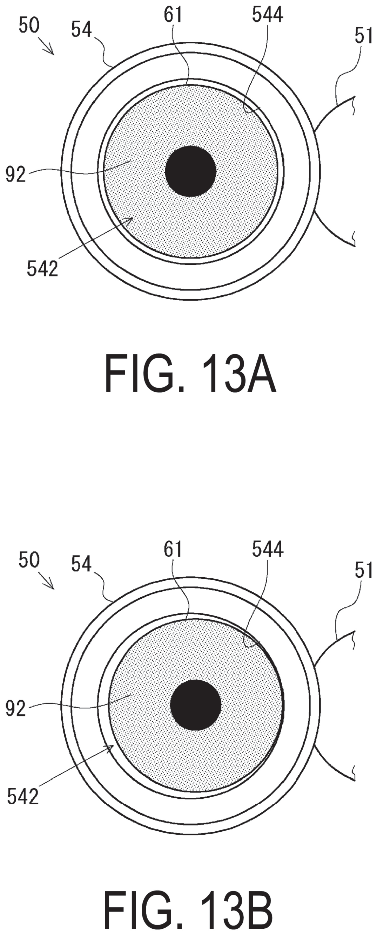

The guide 54 of the intravitreal administration tool 50 is disposed with the guide portion 544 being along the outer edge of the cornea of the eyeball, which is the injection target, during use of the injector 1. FIG. 13A illustrates a state in which the guide 54 is disposed in a manner that an interval between the guide portion 544 and an outer edge 61 of the cornea is uniform. Since the intravitreal administration tool 50 is connected with the guide 54 and the positioning portion 51 having the predetermined positional relationship, when the guide portion 544 is disposed in alignment with the position of the cornea as illustrated in FIG. 13A, the position of the injector tip to which the positioning portion 51 is attached is also determined. In the present embodiment, when the guide 54 is disposed in alignment with the position of the cornea as illustrated in FIG. 13A, the ejection port 77 of the injector 1 is positioned at a position away from the outer edge of the cornea by 3.5 mm to 4.0 mm. Further, when the surface on the distal end side of the guide portion 544 is brought into contact with the eyeball over the entire circumference, the center axis 59 of the positioning portion 51 is at a predetermined angle with respect to the eyeball, and thus the ejection direction of the ejection solution by the injector 1 attached to the positioning portion 51 is also determined. Note that, in FIG. 13A, the guide 54 is disposed in a manner that the interval between the guide portion 544 and the outer edge 61 of the cornea is uniform, but the configuration is not limited thereto, and the guide 54 may be disposed in a manner that a part of the guide portion 544 coincides with the outer edge 61 of the cornea. FIG. 13B illustrates a state in which the guide 54 is disposed in a manner that a portion of the guide portion 544 closest to the positioning portion 51 coincides with the outer edge 61 of the cornea. By disposing the guide 54 as illustrated in FIG. 13B, the guide 54 and the positioning portion 51 may be formed in a manner that the ejection port 77 of the injector 1 is positioned at a position away from the outer edge of the cornea by 3.5 mm to 4.0 mm.

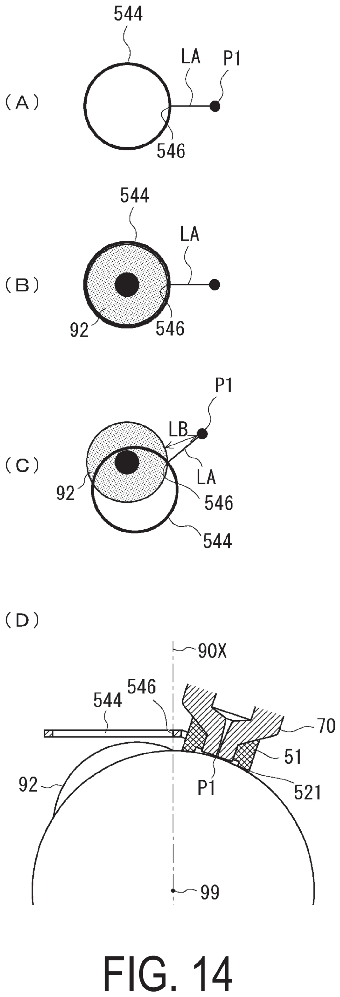

FIG. 14 is a schematic explanatory view illustrating an injection position when the guide 54 is disposed in alignment with the outer edge of the cornea. FIG. 14A illustrates a positional relationship between the guide portion 544 of the guide 54 and an injection position P1. The guide portion 544 has a circular shape, and a starting point when the interval LA from the guide portion 544 to the injection position P1 is measured is indicated as a length measurement starting point 546.

As illustrated in FIG. 14B, when the guide portion 544 of the intravitreal administration tool 50 is disposed along the outer edge of a cornea 92, the length measurement starting point 546 is located on the outer edge of the cornea 92, and the injection position P1 is disposed in the normal direction of the outer edge of the cornea from the length measurement starting point 546. At this time, the shortest distance between the outer edge of the cornea and the injection position P1 is the length of the interval LA.

In contrast, as illustrated in FIG. 14C, when the guide portion 544 of the intravitreal administration tool 50 is disposed not along the outer edge of the cornea 92, even when the length measurement starting point 546 is located on the outer edge of the cornea 92, the injection position P1 is not disposed in the normal direction of the outer edge of the cornea from the length measurement starting point 546. At this time, a shortest distance LB between the outer edge of the cornea and the injection position P1 is not equal to the length of the interval LA, and the distance from the injection position P1 to the outer edge of the cornea might be less than 3.5 mm. Therefore, the intravitreal administration tool 50 of the present embodiment is disposed in a manner that the circular guide portion 544 is along the outer edge of the cornea. Note that when the guide portion 544 of the guide 54 is disposed in alignment with the outer edge of the cornea, the guide portion 544 is not limited to being in contact with the outer edge of the cornea. For example, as illustrated in FIG. 14D, when the guide 54 is disposed away from the surface of the eyeball in the radial direction of the eyeball, the guide portion 544 and the outer edge of the cornea may be disposed overlapping each other when viewed in the axial direction of a center axis 90X passing through the length measurement starting point 546 and a center of the eyeball 99.

Further, when the intravitreal administration tool 50 is pressed against the eyeball, contact surfaces 523, 533 on the distal end side of the protruding portions 521, 531 of the irregular portion 52, 53 come into contact with the eyeball. Therefore, the contact surfaces 523, 533 of the protruding portions 521, 531 are formed along a predetermined curved surface corresponding to the eyeball. Here, the predetermined curved surface is, for example, a spherical surface. By forming the contact surfaces 523, 533 along a spherical surface of an eyeball having an average diameter, the intravitreal administration tool 50 can be used for general purposes. Further, the contact surfaces 523, 533 may be formed along a spherical surface of an eyeball having a diameter slightly larger than the average diameter. In this case, when the intravitreal administration tool 50 is pressed against the eyeball, the inner sides of the contact surfaces 523, 533 may mainly come into contact with the eyeball in a case where the eyeball has a diameter equal to or smaller than the average diameter, and the outer sides of the contact surfaces 523, 533 may mainly come into contact with the eyeball in a case where the eyeball has a diameter larger than the average diameter. Note that the predetermined curved surface along which the contact surfaces 523, 533 are formed is not limited to the spherical surface, and may be a curved surface that deviates from the spherical surface, as long as the contact surfaces 523, 533 of the protruding portions 521, 531 come into contact with the eyeball. For example, the contact surfaces 523, 533 may be formed along an aspherical surface having a high curvature on the axis 58, 59 side (inner side) and a low curvature on the side away from the axis 58, 59 (outer side) in the radial direction of the intravitreal administration tool 50.

As described above, according to the present embodiment, when the guide 54 is disposed along the outer edge of the cornea, the ejection port 77 of the injector 1, from which the substance to be injected is ejected, is positioned at the position away from the outer edge of the cornea by the predetermined distance rearward of the eyeball. Accordingly, the intravitreal administration tool 50 of the present embodiment can avoid damaging the cornea or the crystalline lens due to the injection position being too close to the cornea, and damaging the retina due to the injection position being too far from the cornea, and can provide a technique for facilitating an intravitreal injection.

Further, when the guide 54 is disposed along the outer edge of the cornea, the intravitreal administration tool 50 positions the ejection port 77 of the injector 1 at the position away from the outer edge of the cornea by 3.5 mm to 4.0 mm, and it is thus possible to easily perform a precise intravitreal injection in compliance with the guideline.

Further, when the intravitreal administration tool 50 is pressed against the eyeball, the irregular portions 52, 53 come into contact with the surface of the eyeball and increase the frictional force as compared with a case where the tip of the injector 1 is simply pressed against the eyeball, and thus the misalignment of the injector 1 with respect to the target region of the eyeball can be suppressed.

### Second Embodiment

The present embodiment is different from the first embodiment described above in that an intravitreal administration tool 50A and the container 70 are integrally formed. Note that since other portions are the same as those of the first embodiment described above, the same elements are denoted by the same reference signs and description thereof will not be repeated.

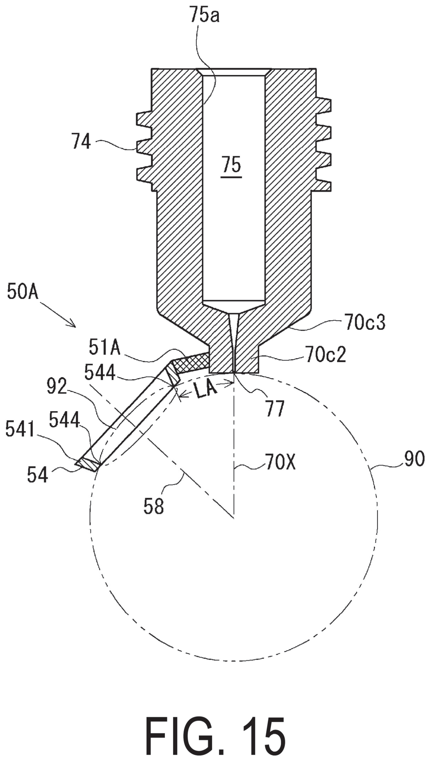

FIG. 15 is a diagram illustrating a schematic configuration of the intravitreal administration tool 50A and the container 70 according to a second embodiment. The intravitreal administration tool 50A includes the guide 54 and a positioning portion 51A and is integrally formed with the container 70. The positioning portion 51A is a columnar member that connects a side portion of the guide 54 and a side portion of the container 70. The positioning portion 5 1A connects the guide 54 and the container 70 in a manner that the guide 54 and the container 70 have a predetermined positional relationship with each other. For example, the positioning portion 51A is provided at a position at which a center axis 70X of the distal end portion 70c2 of the container 70 is away from the guide portion 544 of the guide 54 by the predetermined distance LA.

Further, the positioning portion 51A is formed in a manner that the center axis 70X of the container 70 is at a predetermined angle with respect to the guide 54. For example, the positioning portion 51 connects the guide 54 and the container 70 in a manner that the center axis 70X of the container 70 passes through the eyeball center or the back side (retinal side) relative to the center of the eyeball when the guide 54 is disposed along the outer edge of the cornea.

As described above, in the intravitreal administration tool 50A of the present embodiment, when the guide 54 is disposed along the outer edge of the cornea, the ejection port 77 is positioned at the position away from the outer edge of the cornea by the predetermined distance rearward of the eyeball. Accordingly, the injector 1 provided with the intravitreal administration tool 50A of the present embodiment can avoid damaging the cornea or the crystalline lens due to the injection position being too close to the cornea, and damaging the retina due to the injection position being too far from the cornea, and can provide a technique for facilitating the intravitreal injection.

### Third Embodiment

The present embodiment is different from the first embodiment described above in that a container holder 71 that holds a container 70 is provided and an intravitreal administration tool 50B is attached to the container holder (holding portion) 71. Note that since other portions are the same as those of the first embodiment described above, the same elements are denoted by the same reference signs and description thereof will not be repeated.

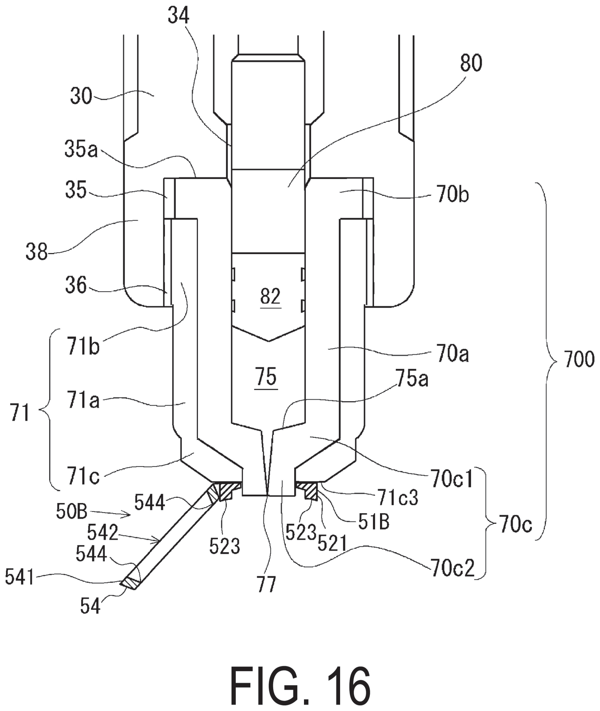

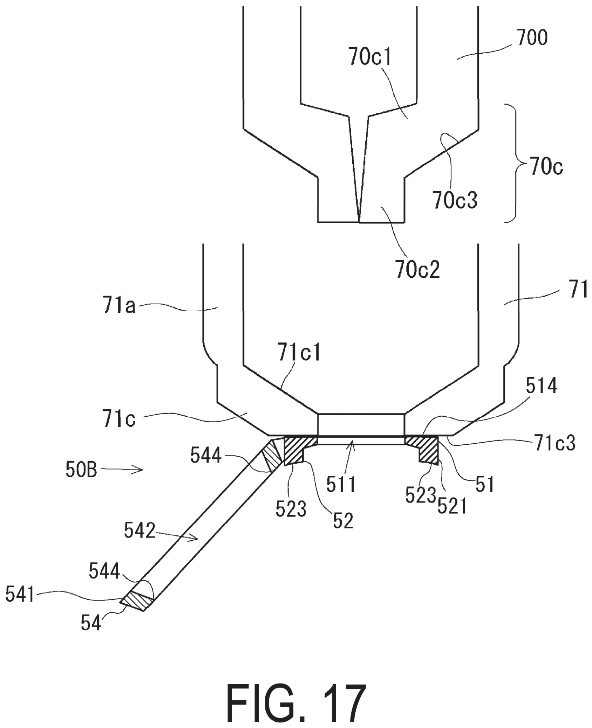

FIG. 16 is a diagram illustrating a schematic configuration of the intravitreal administration tool 50B and a container 700 according to a third embodiment. FIG. 17 is a diagram for describing a positional relationship between the container 700 and the container holder 71 according to the third embodiment.

The container 70B itself of the present embodiment does not include a male thread portion that can be screwed into the female thread portion 36 formed on the inner wall on the distal end side of the attachment 30. Thus, the container 70B is attached to the attachment 30 with use of the container holder 71. The container holder 71 includes a body portion 71a that defines a space containing the container 70B, a mating portion 71b that is connected to the base end side of the body portion 71a and is formed with a male thread that is screwed into the female thread portion 36, and a contact portion 71c that is connected to the distal end side of the body portion 71a and includes an inner surface 71c1 (see FIG. 17) that comes into surface contact with the distal end side outer surface 70c3 of the container 70B. Further, as illustrated in FIG. 17, the contact portion 71c is provided with an opening 71c2 through which the distal end portion 70c2 of the container 700 can pass.

A positioning portion 51B of the intravitreal administration tool 50B is attached to the distal end surface 71c3 of the container holder 71. The intravitreal administration tool 50B includes the positioning portion 51B that is disposed around the axis 59 along the ejection direction of the ejection solution and surrounds the periphery of the distal end portion 70c2, the guide 54 connected to the positioning portion 51B, and the irregular portions 52, 53 provided on the distal end side of the positioning portion 51B and the guide 54, respectively. A surface (base end surface) 514 on the base end side of the positioning portion 51B is attached to the distal end surface 71c3 of the container holder 71 by adhesion, welding, or the like. Note that the intravitreal administration tool 50 of the present embodiment is formed separately from the container holder 71, but may be formed integrally with the container holder 71.

The container 700 is housed with the distal end portion 70c2 passing through the opening 71c2 of the container holder 71 having the above-described configuration. At this time, the container 700 is in a state where the housing space 75 is filled with the substance to be injected and the plunger 80 is loaded. In this housed state, the distal end side outer surface 70c3 of the container 700 and the inner surface 71c1 of the container holder 71 are in surface contact with each other on the distal end side, and the base end portion 70b of the container 700 and the mating portion 71b of the container holder 71 are in contact with each other on the base end side. Then, while the container 700 is housed in the container holder 71, the male thread formed at the mating portion 71b of the container holder 71 and the female thread portion 36 of the attachment 30 are screwed together until the base end side end surface of the base end portion 70b of the container 700 comes into contact with an end surface 35a of a third region 35 of the attachment 30. As a result, with the fastening force produced by the screwing, the container 700 is attached to the attachment 30 with the distal end side outer surface 70c3 of the container 700 pressed by the container holder 71 from the distal end side toward the base end side, that is, in a direction opposite to a movement direction of the plunger 80. At this time, the plunger 80 is in a state of being disposed inside both the housing space 75 in the container 700 and the region where the plunger 80 is generally disposed in the attachment 30.

The steps of assembling the injector assembly 10 by attaching the actuator 20 to the attachment 30 in this state, and the like, and the steps of assembling the injector 1 are the same as those of the first embodiment described above.

As described above, the injector 1 of the present embodiment includes the intravitreal administration tool 50B at the distal end of the container holder 71, and when the guide 54 is disposed along the outer edge of the cornea, the ejection port 77 of the injector 1, from which the substance to be injected is ejected, is positioned at the position away from the outer edge of the cornea by the predetermined distance rearward of the eyeball. Accordingly, the intravitreal administration tool 50 of the present embodiment can avoid damaging the cornea or the crystalline lens due to the injection position being too close to the cornea, and damaging the retina due to the injection position being too far from the cornea, and can provide a technique for facilitating the intravitreal injection.

### Fourth Embodiment

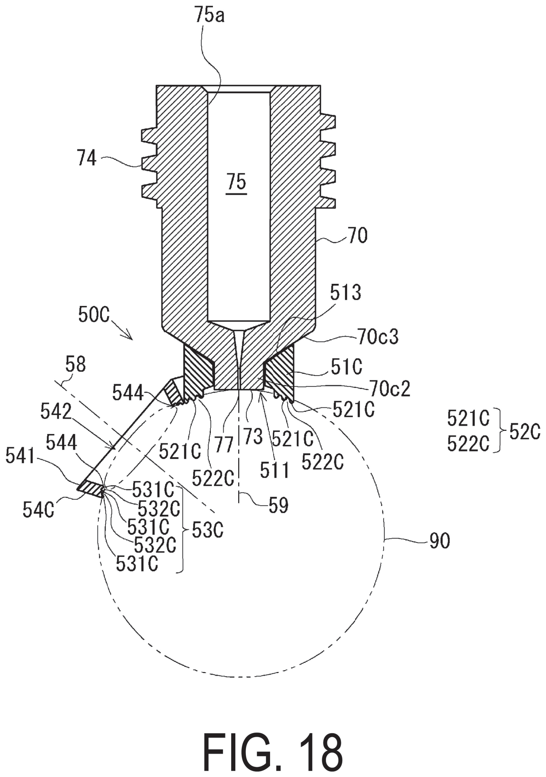

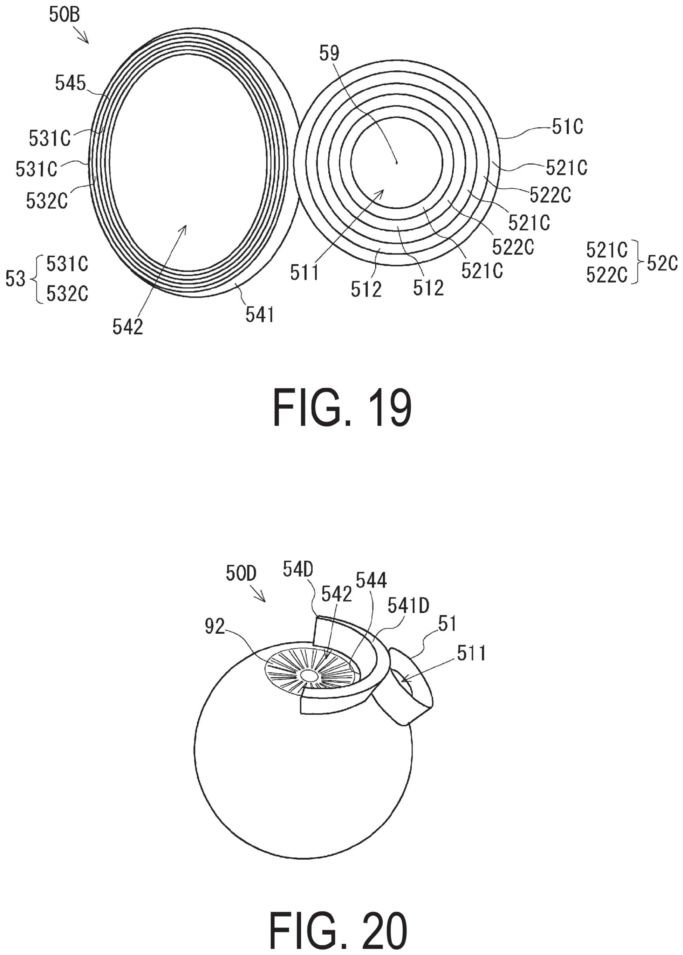

The present embodiment is different from the first embodiment described above in the configuration of irregular portions 52C, 53C. Note that since other portions are the same as those of the first embodiment described above, the same elements are denoted by the same reference signs and description thereof will not be repeated. FIG. 18 is a cross-sectional view illustrating a schematic configuration of an intravitreal administration tool 50C and the container 70 according to a fourth embodiment, and FIG. 19 is a view of the intravitreal administration tool 50C as viewed from the distal end side.

As illustrated in FIGS. 18 and 19, the intravitreal administration tool 50C of the present embodiment includes a positioning portion 51C surrounding the ejection port 77, the guide 54 connected to the positioning portion 51C, and the irregular portions 52C, 53C provided on the target region side of the positioning portion 51C and the guide 54, respectively.

The irregular portion 52C is formed by concentrically erecting a plurality of protruding portions 521C on the surface (base surface) 512 facing the target region of the positioning portion 51C. The protruding portions 521C are provided at intervals in the radial direction of the base surface 512. Accordingly, a recessed portion 522C is formed between the protruding portions 521C. Note that, in the present embodiment, the intervals between the corresponding protruding portions 521C are equal, and the protruding portions 521C are arranged in rotational symmetry about the axis 59. Each of the protruding portions 521C is formed in a circular shape when viewed from the distal end side, but may be provided being divided into a plurality of portions and thus have a shape in which a part of a circle is missing.

Further, the irregular portion 53C is formed by concentrically erecting a plurality of protruding portions 531C on the surface (base surface) 545 facing the target region of the guide 54. The protruding portions 531C are provided at intervals in the radial direction of the base surface 545. Accordingly, a recessed portion 532C is formed between the protruding portions 531C. Note that, in the present embodiment, the intervals between the corresponding protruding portions 531C are equal, and the protruding portions 531C are arranged in rotational symmetry about the axis 58. Each of the protruding portions 531C is formed in a circular shape when viewed from the distal end side, but may be provided being divided into a plurality of portions and thus have a shape in which a part of a circle is missing.

As described above, according to the present embodiment, when the ejection port 77 is pressed against the eyeball during use of the injector 1, the irregular portions 52C, 53C of the intravitreal administration tool 50C come into contact with the surface of the eyeball and increases the frictional force as compared with the case where the ejection port 77 is simply pressed against the eyeball. Thus, the misalignment of the injector 1 with respect to the eyeball can be suppressed, and a technique for further facilitating the intravitreal injection can be provided.

### Fifth Embodiment

The present embodiment is different from the first embodiment described above in the configuration of a guide 54D. Note that since other portions are the same as those of the first embodiment described above, the same elements are denoted by the same reference signs and description thereof will not be repeated.

FIG. 20 is a diagram illustrating a schematic configuration of an intravitreal administration tool 50D according to a fifth embodiment. As illustrated in FIG. 20, in the intravitreal administration tool 50D of the present embodiment, a peripheral wall 541D of a guide 54D is formed in a semicircular shape when viewed from the base end side. That is, as compared with the guide 54 of the first embodiment, the guide 54D of the present embodiment has a shape in which a half of the peripheral wall on a side far from the positioning portion 51 is cut out and a half of the peripheral wall on a side connected to the positioning portion 51 is left.

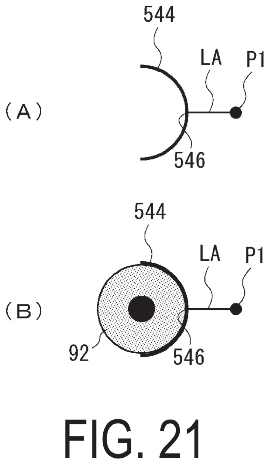

FIG. 21 is a schematic explanatory view illustrating the injection position when the intravitreal administration tool is disposed in alignment with the outer edge of the cornea. FIG. 21A illustrates a positional relationship between the guide portion 544 of the intravitreal administration tool 50D and the injection position P1 in the present embodiment. The guide portion 544 of the present embodiment is semicircular, and the guide portion 544 is disposed along the outer edge of the cornea as in the first embodiment described above. At this time, the guide 54D and the positioning portion 51 are connected to each other in the intravitreal administration tool 50D of the present embodiment in a manner that the injection position P1 is located in the normal direction of the outer edge of the cornea from the length measurement starting point 546. Therefore, when the guide portion 544 of the guide 54D is disposed along the outer edge of the cornea during use of the injector 1, the positioning portion 51 positions the ejection port 77 of the injector 1 at the position away from the outer edge of the cornea by the predetermined distance rearward of the eyeball. Accordingly, the injector 1 provided with the intravitreal administration tool 50D can avoid damaging the cornea or the crystalline lens due to the injection position being too close to the cornea, and damaging the retina due to the injection position being too far from the cornea, and can provide a technique for facilitating the intravitreal injection.

Further, since the guide 54D of the present embodiment has a smaller area in contact with the eyeball than the guide 54 of the first embodiment, even when the eyeball unexpectedly moves while the guide 54D is being pressed against the eyeball, the possibility of the guide 54D damaging the eyeball can be reduced. Further, by downsizing the intravitreal administration tool 50D, handling during an operation is facilitated, and thus the intravitreal injection can be further facilitated. Further, by downsizing the guide 54D, the manufacturing cost, the transportation cost, and the management cost are reduced.

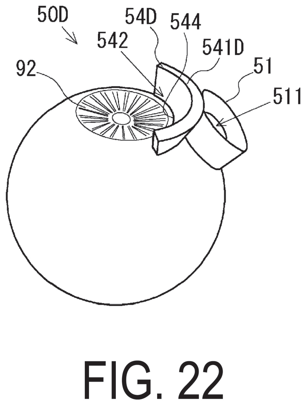

Note that, in the present embodiment, the semicircular guide 54D is provided on the side close to the positioning portion 51, but the guide 54D may be formed on another portion on the circumference surrounding the cornea. Further, the guide 54D is not limited to having the semicircular shape, and may be formed in a manner that at least a part thereof is along the outer edge of the cornea. FIG. 22 is a diagram illustrating a modified example in which the guide 54D has a length in the circumferential direction shorter than that in the example of FIG. 20. By forming the guide 54D to be shorter in this manner, it is possible to further reduce the possibility of damaging the eyeball, to facilitate the handling during an operation, and to reduce the cost, as compared with the example of FIG. 20.

### Modified Examples

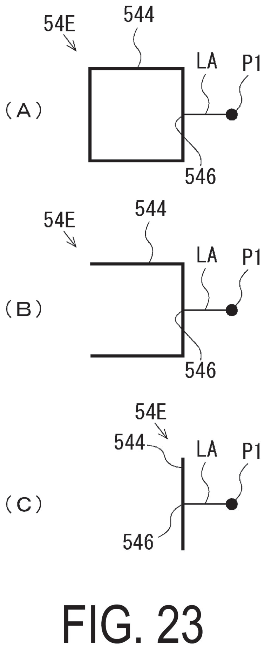

In the embodiments described above, examples of the guide 54, 54D including the curved guide portion 544 are illustrated, but the guide of the intravitreal administration tool is not limited thereto, and may include a linear guide portion. FIG. 23 is a diagram illustrating examples of a guide 54E including a linear guide portion. FIG. 23A illustrates an example in which four linear guide portions 544 are connected to each other to form each side of a rectangle to form the rectangular guide 54E. FIG. 23B illustrates an example in which one side of the rectangle illustrated in A is omitted. Further, FIG. 23C illustrates an example in which the guide 54E is formed in one straight line shape.

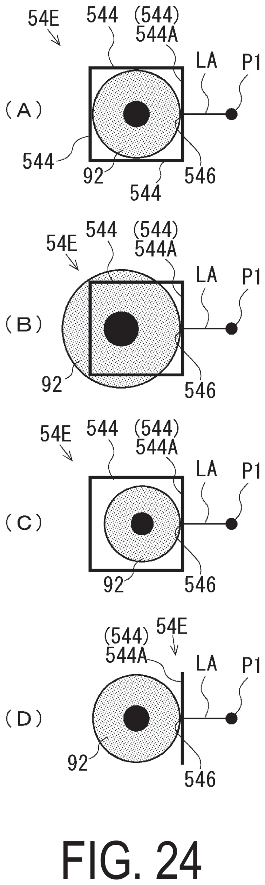

FIG. 24 is a diagram illustrating examples in which the guide portions in FIG. 23 are disposed in alignment with the outer edge of the cornea. As illustrated in FIG. 24A, the guide portion 544 illustrated in FIG. 23A is disposed surrounding the cornea 92. At this time, for example, among the four guide portions 544, at least a guide portion 544A including the length measurement starting point 546 is disposed in alignment with the outer edge of the cornea. Specifically, the guide portion 544A is disposed in contact with the outer edge of the cornea at the position of the length measurement starting point 546 and extends along the tangent of the outer edge of the cornea at the position of the length measurement starting point 546. In this manner, by the linear portion (guide portion 544A) of the guide 54E being disposed along the tangent of the outer edge of the cornea, the injection position P1 is disposed in the normal direction of the outer edge of the cornea from the length measurement starting point 546, and the predetermined interval LA is secured between the outer edge of the cornea and the injection position P1.

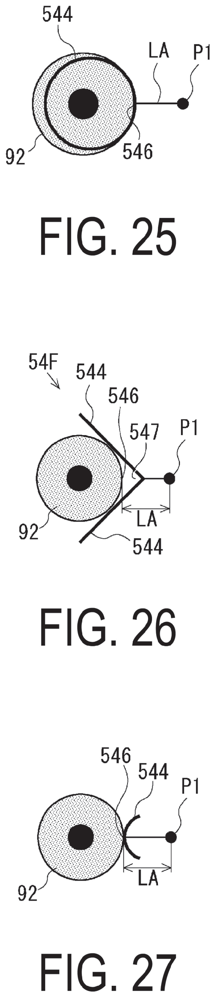

With the guide 54E illustrated in FIG. 23, the linear guide portion 544A is disposed not along the outer edge of the cornea but along the tangent of the outer edge of the cornea, and thus there is an advantage in which positioning is easily performed for various shapes of the cornea. For example, when the circular guide portion 544 is used as illustrated in FIG. 25, when the cornea has a standard diameter, the diameters of the guide portion 544 and the cornea coincide with each other, and thus the positioning can be easily performed. However, when the diameter of the cornea 92 is larger than the diameter of the guide portion 544 as illustrated in FIG. 25, the outer edge of the cornea deviates outward from the guide portion 544 and becomes hidden under the peripheral wall of the guide 54. Thus, it might be difficult to determine whether or not the length measurement starting point 546 is located on the outer edge of the cornea. In contrast, with the guide 54E illustrated in FIG. 23, since the linear guide portion 544A is not formed along the outer edge of the cornea, as illustrated in FIG. 24B, even when the diameter of the cornea is larger than usual, the outer edge of the cornea does not deviate outward from the guide portion 544 when the length measurement starting point 546 is aligned with the outer edge of the cornea, and the positioning is easily performed. Further, as illustrated in FIG. 24C, even when the diameter of the cornea is smaller than usual, the guide 54E can be easily positioned by disposing the guide portion 544A along the tangent of the outer edge of the cornea, as in FIGS. 24A and 24B. Note that the guide portions 544 illustrated in FIGS. 23B and 23C may also be disposed in the same manner. For example, FIG. 24D illustrates an example in which the guide portion 544 illustrated in FIG. 23C is disposed in contact with the outer edge of the cornea at the position of the length measurement starting point 546 and extends along the tangent of the outer edge of the cornea.

Note that, when the guide portion 544A of the guide 54E is disposed in alignment with the outer edge of the cornea, the guide portion 544A is not limited to being in contact with the outer edge of the cornea. For example, as in the case of FIG. 14D described above, when the guide 54E is disposed away from the surface of the eyeball in the radial direction of the eyeball, the guide portion 544A and the outer edge of the cornea may be disposed overlapping each other when viewed in the axial direction of the center axis 90X passing through the length measurement starting point 546 and the center of the eyeball 99.

The guides 54E illustrated in FIG. 23 include the length measurement starting point 546 on the guide portion 544A, but the configuration is not limited thereto, and the length measurement starting point may be set at a position other than the point of contact between the guide portion 544 and the outer edge of the cornea. FIG. 26 is a diagram illustrating an example of a guide 54F in which the length measurement starting point is set at a position other than the point of contact between the guide portion 544 and the outer edge of the cornea. The guide 54F illustrated in FIG. 26 is disposed in a manner that two of the linear guide portions 544 are connected to each other at a predetermined angle and each of the guide portions 544 is in contact with the outer edge of the cornea at one point. At this time, the guide 54F is formed in a manner that, when a point at which a line (bisecting line) bisecting an inner angle 547 formed by the two guide portions 544 intersects with the outer edge of the cornea is defined as the length measurement starting point 546, the injection position P1 is located on a line obtained by extending the bisecting line outward from the inner angle 547. That is, the guide 54F and the positioning portion 51 are connected to each other in a manner that the guide portion 544 and the injection position P1 have the positional relationship illustrated in FIG. 26. Accordingly, the guide 54F is disposed in a manner that each of the two guide portions 544 is in contact with the outer edge of the cornea at one point, whereby the predetermined interval LA is secured between the outer edge of the cornea and the injection position P1.

FIG. 27 is a diagram illustrating an example of the guide portion 544 having a curved shape protruding toward the cornea side. The guide portion 544 illustrated in FIG. 27 has the curved shape but does not have a shape along the outer edge of the cornea, and thus has an advantage in which the positioning is easily performed for various shapes of the cornea, as with the guide 54E illustrated in FIG. 23.

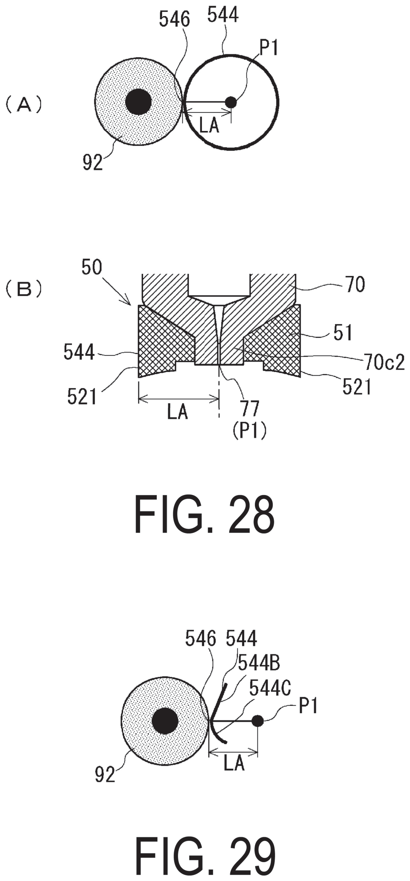

FIG. 28 is a diagram illustrating an example in which the guide portion 544 having a circular shape is disposed surrounding the injection position P1. FIG. 28A schematically illustrates a positional relationship between the guide portion 544 and the injection position P1. The guide portion 544 illustrated in FIG. 28 may be, for example, the outer peripheral surface of the positioning portion 51. FIG. 28B illustrates a cross-sectional shape when the outer peripheral surface of the positioning portion 51 is formed as the guide portion 544. As illustrated in FIG. 28, by forming the positioning portion 51 with the distance from the outer peripheral surface of the positioning portion 51 (intravitreal administration tool) to the ejection port 77 as the predetermined interval LA, the predetermined interval LA is secured between the outer edge of the cornea and the injection position P1 when the outer peripheral surface of the positioning portion 51, which is the guide portion 544, is disposed in contact with the outer edge of the cornea. Accordingly, it is possible to produce the intravitreal administration tool that facilitates the intravitreal injection with a simple configuration.

FIG. 29 is a diagram illustrating an example of the guide portion 544 obtained by combining a straight line and a curved line. The guide portion 544 illustrated in FIG. 29 has a shape including a linear portion 544B and a curved portion 544C. The guide portion 544 illustrated in FIG. 29 is disposed in a manner that the guide portion 544 is in contact with the outer edge of the cornea at the position of the length measurement starting point 546, whereby the predetermined interval LA is secured between the outer edge of the cornea and the injection position P1. Since the guide portion 544 illustrated in FIG. 29 does not have a shape along the outer edge of the cornea, the guide portion 544 has an advantage in which the positioning is easily performed for various shapes of the cornea, as with the guide 54E illustrated in FIG. 23.

Although the embodiments of the intravitreal administration tool 50 according to the present disclosure have been described above, each of the aspects disclosed in the present specification can be combined with any other features disclosed therein.

### Reference Signs List

1 Injector
2 Housing
3 Power cable
4 Connector
5 First switch
6 Second switch
7 Socket
10 Injector assembly
20 Actuator
21 Body
22 Igniter
30 Attachment
40 Piston
50, 50A, 50B, 50C, 50D Intravitreal administration tool
51, 51A, 51B Positioning portion
52, 52C Irregular portion
53, 53C Irregular portion
54, 54D Guide
70, 70B Container
71 Container holder
77 Ejection port
80 Plunger

## Claims

1. An intravitreal administration tool configured to position a needleless injector configured to inject a substance to be injected into a vitreous body of an eyeball without using an injection needle, the intravitreal administration tool comprising:
a guide, at least a part of the guide being formed in a shape corresponding to an outer edge of a cornea of the eyeball; and
a positioning portion connected to the guide and the needleless injector and configured to position an ejection port, of the needleless injector, from which the substance to be injected is ejected, at a position away from the outer edge of the cornea by a predetermined distance rearward of the eyeball when the guide is disposed in alignment with the outer edge of the cornea.

2. The intravitreal administration tool according to claim 1, wherein
the needleless injector includes a housing portion having a housing space in which the substance to be injected is housed, the housing portion including the ejection port from which the substance to be injected is ejected to the eyeball, and
the positioning portion is attached to the housing portion of the needleless injector.

3. The intravitreal administration tool according to claim 1, wherein
the needleless injector includes
a housing portion having a housing space in which the substance to be injected is housed, the housing portion including the ejection port from which the substance to be injected is ejected to the eyeball, and
a holding portion configured to hold the housing portion, and
the positioning portion is attached to the holding portion of the needleless injector.

4. The intravitreal administration tool according to claim 1, wherein
the guide is formed in a curved shape along the outer edge of the cornea.

5. The intravitreal administration tool according to claim 4, wherein
the positioning portion positions the ejection port of the needleless injector at a position away from the outer edge of the cornea by 3.5 mm to 4.0 mm when the guide is disposed along the outer edge of the cornea.

6. The intravitreal administration tool according to claim 1, wherein
the guide is formed in a linear shape in contact with a part of the outer edge of the cornea.

7. The intravitreal administration tool according to claim 4, wherein
the positioning portion positions the ejection port of the needleless injector at a position away from the outer edge of the cornea by 3.5 mm to 4.0 mm when a linear portion of the guide is disposed along a tangent of the outer edge of the cornea.

8. The intravitreal administration tool according to any one of claims 1 to 7, wherein
the positioning portion is provided with an irregular portion on a surface facing the eyeball when the guide is disposed along the outer edge of the cornea, the irregular portion being configured to suppress positional misalignment.

9. The intravitreal administration tool according to any one of claims 1 to 7, wherein
the guide is provided with an irregular portion on a surface facing the eyeball when the guide is disposed along the outer edge of the cornea, the irregular portion being configured to suppress positional misalignment.

10. A needleless injector configured to inject a substance to be injected into a vitreous body of an eyeball without using an injection needle, the needleless injector comprising:
a housing portion having a housing space in which the substance to be injected is housed and an ejection port from which the substance to be injected is ejected to the eyeball;
a drive unit configured to provide ejection energy for ejecting the substance to be injected; and
an intravitreal administration tool configured to determine an injection position of the needleless injector with respect to the eyeball, wherein
the intravitreal administration tool includes
a guide, at least a part of the guide being formed in a shape corresponding to an outer edge of a cornea of the eyeball; and
a positioning portion connected to the guide and the needleless injector and configured to position the ejection port at a position away from the outer edge of the cornea by a predetermined distance rearward of the eyeball when the guide is disposed in alignment with the outer edge of the cornea.

11. The needleless injector according to claim 10, wherein
the positioning portion of the intravitreal administration tool is attached to the housing portion.

12. The needleless injector according to claim 10, further comprising:
a holding portion configured to hold the housing portion, wherein
the positioning portion of the intravitreal administration tool is attached to the holding portion.

13. The needleless injector according to claim 10, wherein
the guide is formed in a curved shape along the outer edge of the cornea.

14. The needleless injector according to claim 13, wherein
the positioning portion positions the ejection port of the needleless injector at a position away from the outer edge of the cornea by 3.5 mm to 4.0 mm when the guide is disposed along the outer edge of the cornea.

15. The needleless injector according to claim 10, wherein
the guide is formed in a linear shape in contact with a part of the outer edge of the cornea.

16. The needleless injector according to claim 15, wherein
the positioning portion positions the ejection port of the needleless injector at a position away from the outer edge of the cornea by 3.5 mm to 4.0 mm when a linear portion of the guide is disposed along a tangent of the outer edge of the cornea.

17. The needleless injector according to any one of claims 10 to 16, wherein
the positioning portion is provided with an irregular portion on a surface facing the eyeball when the guide is disposed along the outer edge of the cornea, the irregular portion being configured to suppress positional misalignment.

18. The needleless injector according to any one of claims 10 to 16, wherein
the guide is provided with an irregular portion on a surface facing the eyeball when the guide is disposed along the outer edge of the cornea, the irregular portion being configured to suppress positional misalignment.
